# EUROPEAN PATENT SPECIFICATION

(11) **EP 2 454 950 B1**
(45) Date of publication and mention of the grant of the patent: **03.09.2014**
(21) Application number: 10799869.2
(22) Date of filing: 14.07.2010
(51) Int. Cl.: A23L 1/214, A23L 1/212, A23L 1/221, A23L 1/30, A23L 2/00, A61K 36/896, A61P 3/02

(54) **ONION EXTRACT, AND PROCESS FOR PRODUCTION THEREOF**
ZWIEBELEXTRAKT UND HERSTELLUNGSVERFAHREN DAFÜR
EXTRAIT D'OIGNON, ET PROCÉDÉ POUR SA PRODUCTION

(30) Priority: 17.07.2009 JP 2009168394
(43) Date of publication of application: 23.05.2012
(73) Proprietor: Takasago International Corporation, Tokyo-to 144-8721 (JP)
(72) Inventor: YONESHIGE, Arata, Hiratsuka-shi, Kanagawa 254-0073 (JP); SAWAMURA, Ryosuke, Hiratsuka-shi, Kanagawa 254-0073 (JP); HIRAMOTO, Tadahiro, Hiratsuka-shi, Kanagawa 254-0073 (JP); YAMAMOTO, Taichi, Ohta-ku 144-8721 Tokyo (JP)
(74) Representative: Uchida, Kenji
(86) International application number: PCT/JP2010/061921
(87) International publication number: WO 2011/007811

(56) References cited:
- WO-A1-2005/070441
- FR-A1- 2 882 933
- JP-A- 3 155 766
- JP-A- 2001 269 148
- JP-A- 2002 051 722
- JP-A- 2002 186 448
- JP-A- 2002 186 455
- JP-A- 2002 253 171
- JP-A- 2005 013 138
- J.R.WHITAKER: 'Development of Flavor, Odor, and Pungency in Onionand Garlic' ADV. FOOD RES. vol. 22, 1976, pages 73 - 133, XP008150693
- KAZUMI SEBE ET AL.: 'Purification and Structure of Antioxidative Compounds from Onion Rinds Extract' JOURNAL OF HEALTH AND SOCIAL WELFARE SCIENCE OF NISHIKYUSHU UNIVERSITY vol. 38, 01 March 2008, pages 17 - 21, XP008150697
- LI-KUN HAN ET AL.: 'Effect of an Aqueous Extract of Onion Skin on the Postprandial Triacylglycerol Level in Rodents' THE JAPANESE JOURNAL OF NUTRITION AND DIETETICS vol. 66, no. 2, 01 April 2008, pages 77 - 81, XP008150694

## Description

### Technical Field

The present invention relates to an onion extract. More specifically, the invention relates to an onion extract which shows no harshness or astringency and can impart the inherent deliciousness and sweetness of onion to foods or drinks. The invention also relates to a process for producing the onion extract having the aforesaid characteristics, and a flavor composition containing the onion extract and a food or drink containing the onion extract.

### Background Art

Onion has been commonly used as, for example, a starting material for preparing dressings, stews, soups and so on, an ingredient for curry and other foods, and a flavor for drinks. By using onion as an ingredient of foods or a flavor for drinks, not only the characteristic flavor of onion but also the sweetness thereof are imparted so that delicious foods or drinks can be provided.

When producing the aforesaid foods or drinks in a commercial scale, onion extracts are frequently used as a substitute for fresh onions from the standpoint of working efficiency. As discussed above, onion and onion extracts are added to foods or drinks as a functional food material capable of imparting a taste to foods or drinks. In addition, these materials are used as an ingredient of liquid seasonings. Further, onion extracts are used as an ingredient of health foods, drugs, insecticides, soil-improving agents, bactericides and so on.

To produce an onion extract from fresh onions, various processes have been proposed hitherto. Some of these processes will be provided hereinbelow by way of example.

Examples of these processes include: a process which includes cutting onions into small pieces, optionally adding an oil or fat, roasting the resultant mixture, adding water to the roasted onions, extracting soluble components therefrom at 60 to 100°C, press-filtering the extract, and removing insoluble components therefrom by, for example, centrifugation, optionally followed by concentration, if necessary; a process which includes cutting onions into small pieces, adding water thereto and heating to thereby extract soluble components, and press-filtering the extract optionally followed by concentration, if necessary (refer to Patent Document 1); and a process which includes treating onions at a temperature of - 15 to -25°C over a sufficient period of time for destructing cell membrane, extracting the onions with water or an alcoholic solvent, and then removing the solvent from the extract (refer to Patent Document 2).

Further, examples of the processes include: a process which includes extracting onions with an alcoholic solvent and then removing the solvent from the extract (refer to Patent Documents 3 and 4); and a process for producing an onion extract which includes treating onions with the use of at least one enzyme selected from the group consisting of a carbohydrase and a nuclease together with a proteinase (refer to Patent Document 5).

Further, examples thereof include a process for producing an onion extract which includes blanching peeled onions and then treating the onions with a carbohydrase (refer to Patent Document 6); and a process for producing an onion extract which includes extracting onions with an aqueous alcohol at a low temperature of -25 to 5°C (refer to Patent Document 7).

Furthermore, examples thereof include a process for producing an onion extract which includes using peeled fresh onions as a starting material in an unheated state, either as such or after cutting to a minimum extent, and adding water together with cell wall-destructing enzyme(s) such as protopectinase combined with pectinase or cellulase to thereby conduct an enzymatic reaction (refer to Patent Document 8).

### Prior Art Documents

### Patent Documents

Patent Document 1: JP-A-3-155766
Patent Document 2: JP-A-8-10503
Patent Document 3: JP-A-2002-186448
Patent Document 4: JP-A-2002-186449
Patent Document 5: JP-A-2003-102417
Patent Document 6: JP-A-2004-33022
Patent Document 7: JP-A-2004-357650
Patent Document 8: JP-A-2008-61589

Other patent documents are WO-A-2005/070441, FR-A-2882933, JP-A-2001-269148, JP-A-2002-186455, JP-A-2002-051722, JP-A-2002-253171, JP-A-2005-013138. Non-patent documents XP008150693, XP008150697, and XP008150694.

### Summary of the Invention

### Problems that the Invention is to Solve

However, onion extracts obtained by the conventional processes have harshness and astringency. In addition, these onion extracts are unsatisfactory in the inherent sweetness, deliciousness and richness of onion. In particular, these onion extracts are still insufficient in the capability of imparting the inherent sweetness and deliciousness of onion without showing harshness or astringency to foods or drinks.

Accordingly, an object of the invention is to provide an onion extract which is free from such harshness or astringency as in the conventional onion extracts, shows higher deliciousness and sweetness inherent to onion compared with the conventional onion extracts, and can impart the aforesaid excellent characteristics, even in a small amount, to foods or drinks.

Another object of the invention is to provide a process for producing the onion extract which has the aforesaid preferable characteristics.

Another object of the invention is to provide a flavor composition which includes the onion extract having the aforesaid preferable characteristics.

Another object of the invention is to provide a food or drink which contains the onion extract having the aforesaid preferable characteristics or the flavor composition as described above.

### Means for Solving the Problems

To solve the aforesaid problems, the inventors have conducted extensive studies and discussions and, as a result, found out that one of the active ingredients controlling the sweetness and deliciousness of cooked onion is glutamylmethionine (hereinafter sometimes abbreviated as "GM").

They have also found out that a substance causing "harshness/astringency" in the conventional onion extracts constitutes of two components of quercetin (hereinafter sometimes abbreviated as "QC") and protocatechuic acid (hereinafter sometimes abbreviated as "PA").

Furthermore, the inventors have found out that a material, which is capable of imparting the excellent flavor and deliciousness of onion without showing the aforesaid harshness or astringency in the case of adding to foods or drinks, can be obtained when the weight ratio of glutamylmethionine to quercetin and the weight ratio of glutamylmethionine to protocatechuic acid fall within specific ranges, respectively and, as a preferred embodiment, the concentrations thereof in solid matters of the onion extract are regulated within definite ranges, respectively.

The invention has been completed based on these findings. The onion extract thus obtained is applicable to various processed foods such as liquid seasonings and sauces to thereby give processed foods having the inherent sweetness and deliciousness of onion without showing harshness or astringency.

Accordingly, the invention relates to the following onion extract, a process for producing the onion extract, a flavor composition containing the onion extract and a food or drink containing the same.

1. An onion extract which contains glutamylmethionine, quercetin and protocatechuic acid, wherein a weight ratio of the quercetin to the glutamylmethionine (quercetin/glutamylmethionine) is 0 to 0.49 and a weight ratio of the protocatechuic acid to the glutamylmethionine (protocatechuic acid/glutamylmethionine) is 0 to 1.86.
2. The onion extract according to the above 1, wherein a concentration of the glutamylmethionine in an extract solid matter is 350 ppm or more, a concentration of the quercetin in the extract solid matter is 170 ppm or less, and a concentration of the protocatechuic acid in the extract solid matter is 650 ppm or less.
3. The onion extract according to the above 1 or 2, which is obtained by a production process comprising: any one of the following steps (a) to (d); and a step of distribution washing a crude onion extract, onion suspension or concentrate thereof, which has been obtained by the following step, with an organic solvent,
   (a) a step of heating the crude onion extract or concentrate thereof, which is obtained by at least either extracting or squeezing a fresh onion;
   (b) a step of heating a fresh onion, and then obtaining the crude onion extract or concentrate thereof by at least either extracting or squeezing;
   (c) a step of heat-extracting a fresh onion, thereby giving the crude onion extract or concentrate thereof; and
   (d) a step of heating a fresh onion and then cutting into small pieces, thereby giving the onion suspension or concentrate thereof.
4. The onion extract according to the above 3, wherein the organic solvent used in the distribution washing is at least one of ethyl acetate and ethanol.
5. A process for producing an onion extract, the process comprising: any one of the following steps (a) to (d); and a step of distribution washing a crude onion extract, onion suspension or concentrate thereof, which has been obtained by the following step, with an organic solvent, wherein the onion extract contains glutamylmethionine, quercetin and protocatechuic acid, and a weight ratio of the quercetin to the glutamylmethionine (quercetin/glutamylmethionine) is 0 to 0.49 and a weight ratio of the protocatechuic acid to the glutamylmethionine (protocatechuic acid/glutamylmethionine) is 0 to 1.86,
   (a) a step of heating the crude onion extract or concentrate thereof, which is obtained by at least either extracting or squeezing a fresh onion;
   (b) a step of heating a fresh onion, and then obtaining the crude onion extract or concentrate thereof by at least either extracting or squeezing;
   (c) a step of heat-extracting a fresh onions, thereby giving the crude onion extract or concentrate thereof; and
   (d) a step of heating a fresh onion and then cutting into small pieces, thereby giving the onion suspension or concentrate thereof.
6. The production process according to the above 5, wherein the organic solvent used in the distribution washing is at least one of ethyl acetate and ethanol.
7. A flavor composition, which contains 0.01 to 50 wt% of the onion extract according to any of the above 1 to 4.
8. A food or drink, which contains 0.01 to 10 wt% of the flavor composition according to the above 7.
9. A food or drink which contains 0.00001 to 10 wt% of the onion extract according to any of the above 1 to 4.

### Advantages of the Invention

In the case of adding to foods or drinks or using in foods or drinks, the onion extract of the invention is capable of imparting the inherent umami and sweetness of onion to the foods or drinks without showing the aforesaid harshness or astringency, compared with the conventional onion extracts.

According to the process for producing the onion extract of the invention, an onion extract having improved qualities can be easily produced using a marketed onion extract.

According to the process for producing the onion extract of the invention, moreover, the weight ratios of glutamylmethionine, quercetin and protocatechuic acid and the concentrations thereof in the solid matters in the onion extract can be regulated within a specific range by simple procedures, i.e., heating and treating with an organic solvent in the course of obtaining an conventional onion extract. Thus, the onion extract, which is capable of imparting the inherent umami and sweetness of onion to foods or drinks without showing the harshness or astringency observed in the conventional onion extracts, can be produced.

### Brief Description of the Drawings

[Fig. 1] Fig. 1 is a cobweb chart showing the results of the evaluation in 7 items (sweetness, umami, richness, astringency, bitterness, sourness and burnt flavor) of the onion extracts obtained in Example 6 and Comparative Examples 5 to 7.
[Fig. 2] Fig. 2 is a cobweb chart showing the results of the evaluation in 7 items (sweetness, umami, richness, astringency, bitterness, sourness and burnt flavor) of the onion extracts obtained in Example 7 and Comparative Examples 8 to 10.

### Mode for Carrying Out the Invention

Next, the modes for carrying out the invention will be described in detail.

In the onion extract of the invention, the weight ratio of quercetin to glutamylmethionine (quercetin/glutamylmethionine) is 0 to 0.49. The weight ratio thereof is preferably 0.40 or less, still preferably 0.37 or less, still more preferably 0.16 or less and particularly preferably 0.12 or less. Although the lower weight ratio is the better, the weight ratio of 10⁻⁸ or more is usually preferred.

In the onion extract of the invention, the weight ratio of protocatechuic acid to glutamylmethionine (protocatechuic acid/glutamylmethionine) is 0 to 1.86. The weight ratio thereof is preferably 1.71 or less, still preferably 1.52 or less, still more preferably 1.09 or less and particularly preferably 0.91 or less. Although the lower weight ratio is the better, the weight ratio of 10⁻¹⁰ or more is usually preferred.

By controlling the weight ratio of quercetin/glutamylmethionine and the weight ratio of protocatechuic acid/glutamylmethionine in the onion extract to the above ranges, an onion extract, which is capable of imparting the inherent umami and sweetness of onion to foods or drinks without showing the harshness or astringency observed in the conventional onion extracts, can be obtained.

The onion extract of the invention may be produced by an arbitrary process, so long as it satisfies the aforesaid weight ratios. When the onion extract of the invention is produced, the onion extract of the invention can be produced from fresh onions.

Alternatively, the onion extract of the invention can be also produced by using a marketed onion extract (hereinafter sometimes referred to as "a starting crude onion extract"). Such a marketed onion extract (i.e., a starting crude onion extract) corresponds to a crude onion extract of the invention or a concentrate thereof.

In the solid matters in the onion extract of the invention, the concentration of glutamylmethionine is preferably 350 ppm or more and still preferably 400 ppm or more. The higher concentration thereof is the better. Although there is no upper limit for the glutamylmethionine concentration, the concentration of 99.9 wt% or less is usually preferred.

By controlling the glutamylmethionine concentration in the solid matters in the onion extract of the invention to 350 ppm or more, the sweetness, umami and richness of onion can be imparted to the onion extract.

In the solid matters in the onion extract of the invention, the concentration of quercetin is preferably 170 ppm or less and still preferably 100 ppm or less. The lower concentration thereof is the better. Although there is no lower limit for the quercetin concentration, the concentration of 0.01 ppm or more is usually preferred.

In the solid matters in the onion extract of the invention, the concentration of protocatechuic acid is preferably 650 ppm or less and still preferably 560 ppm or less. The lower concentration thereof is the better. Although there is no lower limit for the protocatechuic acid concentration, the concentration of 0.001 ppm or more is usually preferred.

By controlling the quercetin concentration and the protocatechuic acid concentration in the solid matters in the onion extract of the invention to 170 ppm or less and 650 ppm or less, respectively, the harshness and astringency can be relieved. In the onion extract of the invention, quercetin and protocatechuic acid are optional ingredients.

As the starting material for producing the onion extract of the invention, fresh onions may be used. To easily obtain the onion extract of the invention, it is also possible to use a marketed onion extract (i.e., a starting crude onion extract).

In the case of producing the onion extract from the fresh onions by a conventional process, however, the obtained onion extract contains quercetin and protocatechuic acid at high concentrations in the solid matters thereof while the concentration of glutamylmethionine is low. The same applies to the marketed onion extracts.

That is to say, onion extracts produced by the conventional processes and marketed onion extracts do not satisfy the requirements relating to the weight ratio of quercetin/glutamylmethionine and the weight ratio of protocatechuic acid/glutamylmethionine as specified in the onion extract of the invention.

As described above, quercetin and protocatechuic acid cause the harshness and astringency of onion extract. To obtain an onion extract showing no harshness or astringency, therefore, it is preferred to reduce the contents of these ingredients. In contrast, it is preferred to increase the concentration of glutamylmethionine in the solid matters in the onion extract.

To increase the glutamylmethionine concentration in the solid matters in the onion extract, the most simple process includes adding glutamylmethionine itself, to the onion extract. However, this process is not necessarily preferred, since glutamylmethionine itself is expensive.

Another process for increasing the glutamylmethionine concentration in the solid matters in the onion extract includes employing a heating treatment at any point in the process of producing the onion extract of the invention from fresh onions. Thus, the concentration of glutamylmethionine in the solid matters in the onion extract can be increased.

The heating treatment may be conducted when fresh onions are extracted. Alternatively, a liquid obtained by at least either extracting or squeezing fresh onions may be heated. Needless to say, fresh onions may be heated before at least either extracting or squeezing.

The liquid, which is obtained by at least either extracting or squeezing fresh onions, may be heated in a concentrated state. Alternatively, it may be heated before concentration. Examples of the procedure for the concentration include vacuum concentration, heat concentration, freeze concentration and membrane concentration.

Fresh onions may be directly heated by, for example, frying the onions themselves. In this case, oil may be added before heating. As the oil, use can be made of those commonly employed in the art, for example, an animal oil or a vegetable oil.

It is preferable to conduct the heating usually at 60°C or higher, still preferably at 70°C or higher and particularly preferably at 80°C or higher. It is usually preferable that the heating temperature is 300°C or lower. The heating is conducted usually for 5 minutes to 12 hours, still preferably for 30 minutes to 6 hours and particularly preferably for 1 to 3 hours.

At a higher heating temperature, the heating can be completed within the shorter period of time. However, by controlling the heating temperature not to exceed the upper limit as described above, it can be prevented from scorching. By conducting the heating over a long period of time, the heating effect can be achieved even at a low temperature. From the stand point of working efficiency, however, it is preferred to control the heating temperature to a level higher than the lower limit as described above.

As the process for lowering the concentrations of quercetin and protocatechuic acid in the onion extract, it is appropriate to employ a process in which quercetin and protocatechuic acid are selectively removed from the solid matters in a crude onion extract or an onion suspension without removing components other than quercetin and protocatechuic acid as far as possible, by conducting distribution washing with the use of an organic solvent (hereinafter sometimes referred to as "solvent-distribution washing").

Needless to say, the process for lowering the quercetin and protocatechuic acid concentrations is not restricted to the distribution washing treatment with the use of an organic solvent.

The term "distribution washing" as used herein means a procedure in which a crude onion extract or onion suspension is contacted with an organic solvent and, after the contact treatment, the organic solvent is removed.

The weight ratios of quercetin to glutamylmethionine and protocatechuic acid to glutamylmethionine can be lowered by removing quercetin and protocatechuic acid in the onion extract without removing glutamylmethionine therefrom.

By removing quercetin and protocatechuic acid from the solid matters in the onion extract, the amount of solid matters in the onion extract is reduced, without changing the amount of glutamylmethionine.

Namely, the glutamylmethionine concentration in the solid matters of the onion extract can be relatively increased by removing quercetin and protocatechuic acid in the solid matters in the onion extract without removing glutamylmethionine. Thus, the glutamylmethionine concentration preferred in the invention can be achieved.

As the organic solvent to be used in the distribution washing, an organic solvent, in which quercetin and protocatechuic acid in the crude onion extract or onion suspension are soluble but glutamylmethionine is not soluble, is preferable as discussed above.

Appropriate examples of such organic solvents include ethyl acetate and ethanol. One kind of the solvent or a solvent mixture in which multiple kinds of solvents are mixed may be used.

In the distribution washing, the organic solvent may be used in an arbitrary amount so long as the objects of the invention can be achieved thereby. It is usually preferred to use the organic solvent in an amount 0.01 to 2 times by weight as much as the crude onion extract, concentrate thereof, onion suspension or concentrate thereof.

Although the number of the distribution washing treatments is not particularly restricted, it is usually preferred to carry out the distribution washing 1 to 3 times, still preferably 1 or 2 times, for a solvent of a single composition. The distribution washing treatment may be carried out with a solvent of a single composition alone. Alternatively, solvents of different compositions may be used for the individual treatments.

In the case of subjecting the crude onion extract or onion suspension to the distribution washing treatment with the use of a solvent which is highly miscible with water such as ethanol, the concentration of the crude onion extract or onion suspension in the distribution washing step is preferably Bx. 60 or higher, still preferably Bx. 70 or higher.

By controlling the concentration of the crude onion extract or onion suspension to Bx. 60 or higher in the distribution washing step, the mixing of the crude onion extract or onion suspension with the solvent can be inhibited and, therefore, the solvent can be removed more easily.

As the process for obtaining the crude onion extract which has been neither heated nor treated with a solvent, use can be made of any process known in the art. The crude onion extract may be produced by, for example, any of the processes as described in the section of Background Art.

More specifically, typical processes for obtaining the crude onion extract include the following processes (1) to (5). Needless to say, it is also possible to employ processes other than them.
(1) A process in which a solvent is added to fresh onions, which are directly used or have been cut into small pieces, followed by exracting at an appropriate temperature, and then the insoluble matters are removed to give an extract and, if required, the extraction solvent is removed from the extract by vacuum concentration.

In the process (1), an extraction solvent is added to washed fresh onions that are used either as such or after cutting into small pieces. Preferable examples of the extraction solvent include water and aqueous ethanol. It is preferred that the ethanol concentration of the aqueous ethanol is 70 wt% or lower, still preferably 50 wt% or lower and still preferably 30 wt% or lower.

It is preferred to use the solvent in an amount 0.5 to 20 times by weight as much as the fresh onions, still preferably 1 to 10 times by weight and still more preferably 1 to 3 times by weight.

The extraction temperature is preferably 10 to 55°C. It is preferred that the extraction is carried out for 30 minutes to 12 hours, still preferably 30 minutes to 6 hours and still more preferably 1 to 4 hours.

Next, the insoluble matters are removed and, if necessary, the extract is vacuum concentrated, thereby giving a crude onion extract. Examples of the procedure for removing the insoluble matters include those commonly employed in the art, such as centrifugation, press filtration, paper filtration and filtration using a filter aid.
(2) A process in which fresh onions are pressed and squeezed, followed by removing insoluble matters to give a squeezed juice and, if required, the juice is vacuum concentrated.

According to the process (2), washed fresh onions are pressed and insoluble matters are removed. Then, the squeezed juice thus obtained is vacuum concentrated, if required, thereby giving a crude onion extract. Examples of the procedure for removing the insoluble matters include those commonly employed in the art, such as centrifugation, press filtration, paper filtration and filtration using a filter aid. The fresh onions may be cut into small pieces with a food mixer or the like.
(3) A process in which cell membrane of fresh onions is destructed by, for example, enzymatically treating or freezing, followed by extracting the contents of the cells with a solvent (water and aqueous alcohol, etc.) at an appropriate temperature, and then the extraction solvent is removed from the extract.
(4) A process in which a solvent is added to fresh onions, which are directly used or have been cut into small pieces, followed by extracting at an appropriate temperature, and then the onions used in the extraction is squeezed together with the extraction solvent, followed by removing the insoluble matters to give a squeezed juice and, if required, the solvent is removed from the squeezed juice by vacuum concentration.
(5) A process in which a solvent is added to fresh onions, which are directly used or have been cut into small pieces, followed by extracting at an appropriate temperature, and then the insoluble matters are removed to give an extract, and further the onions used in the extraction are squeezed, followed by removing the insoluble matters to give a squeezed juice, and then the extract is combined with the squeezed juice and, if required, the solvent is removed from the liquid mixture by vacuum concentration.

In the processes (4) and (5), the same extraction conditions and procedure for removing the insoluble matters as employed in the process (1) may be used.

The crude onion extract, which is obtained by any one of the aforesaid processes (1) to (5), is heated. Then, quercetin and protocatechuic acid contained therein are reduced by the distribution washing with the use of an organic solvent and, if required, glutamylmethionine is added. Thus, the onion extract of the invention can be obtained.

Since the distribution washing with the organic solvent as described above is usually carried out as the final step in the process for producing the onion extract of the invention, it is necessary in any case to produce the crude onion extract or onion suspension to be subjected to the distribution washing.

A preferable example of the process for obtaining the onion extract of the invention includes any one of the following steps (a) to (d); and a step for distribution washing the crude onion extract, onion suspension or a concentrate thereof, that has been obtained by the above step, with an organic solvent.
(a) A step of heating a crude onion extract or concentrate thereof, which has been obtained by at least either extracting or squeezing fresh onions.

The step (a) is a step for obtaining a crude onion extract, wherein a liquid, which is obtained at least either by extracting or squeezing fresh onions, is optionally concentrated and then the crude onion extract or concentrate thereof is heated, followed by distribution washing with the use of an organic solvent.

It is usually preferred that the heating temperature is 60°C or higher, still preferably 70°C or higher and particularly preferably 80°C or higher. It is also preferred in usual that the heating temperature is 300°C or lower. It is usually preferred that the heating time is 5 minutes to 12 hours, still preferably 30 minutes to 6 hours and particularly preferably 1 to 3 hours.
(b) A step of heating fresh onions and then obtaining a crude onion extract or a concentrate thereof by at least either extracting or squeezing.

The step (b) is a step for obtaining a crude onion extract that is to be subjected to the distribution washing, which includes heating fresh onions until golden brown, then pressing and squeezing the heated onions, removing the insoluble matters therefrom to give a squeezed juice and, if required, concentrating.

Alternatively, a crude onion extract that is to be subjected to the distribution washing with an organic solvent can be obtained by adding a solvent to heated onions to extract the same, removing the insoluble matters therefrom to give an extract and, if required, concentrating the extract.

Alternatively, a crude onion extract that is to be subjected to the distribution washing with an organic solvent can be obtained by adding a solvent to heated onions to extract the same, squeezing the onions used in the extraction together with the extraction solvent, removing the insoluble matters therefrom to give an onion squeezed juice and, if required, concentrating the same.

Alternatively, a crude onion extract that is to be subjected to the distribution washing with an organic solvent can be obtained by adding a solvent to heated onions to extract the same, removing the insoluble matters to give an extract, pressing and squeezing the onions used in the extraction and removing the insoluble matters to give a squeezed juice, then combining the extract with the squeezed juice and, if required, concentrating the same.

Preferable examples of the solvent to be used in the extraction in the above steps (a) and (b) include water and aqueous ethanol. It is preferred that the ethanol concentration of the aqueous ethanol is 70 wt% or lower, still preferably 50 wt% or lower and particularly preferably 30 wt% or lower.

It is preferred to use the solvent in an amount 0.5 to 20 times by weight as much as the fresh onions [in the step (a)] or the heated onions [in the step (b)], still preferably 1 to 10 times by weight and still more preferably 1 to 3 times by weight.

The extraction temperature is preferably 10°C or higher. It is preferred that the extraction is carried out for 30 minutes to 12 hours, still preferably 30 minutes to 6 hours and still more preferably 1 to 4 hours.
(c) A step of heat-extracting fresh onions, thereby giving a crude onion extract or a concentrate thereof.

Heat-extraction means an extracting treatment which is conducted at an extraction temperature of 60°C or higher and in which extraction and heating are conducted simultaneously and, therefore, no heating is needed after the extraction.

In the step (c), a solvent or heated solvent is added to fresh onions and extraction is conducted under heating. Namely, the heating step also serves as an extraction step and, therefore, no additional heating is required. Then, the insoluble matters are removed to give an extract, and the extract is then concentrated if required, thereby giving a crude onion extract that is to be subjected to the distribution washing.

Alternatively, a crude onion extract that is to be subjected to the distribution washing with an organic solvent can be obtained by, after the heat-extraction, squeezing the onions used in the extraction together with the extraction solvent, removing the insoluble matters to give an onion squeezed juice and, if required, concentrating the same.

Alternatively, a crude onion extract that is to be subjected to the distribution washing with an organic solvent can be obtained by, after the heat-extraction, removing the insoluble matters to give an extract, pressing and squeezing the onions used in the extraction and removing the insoluble matters to give a squeezed juice, then combining the extract with the squeezed juice and, if required, concentrating the same.

Preferable examples of the solvent to be used in the heat-extraction include water and aqueous ethanol. It is preferred that the ethanol concentration of the aqueous ethanol is 70 wt% or lower, still preferably 50 wt% or lower and particularly preferably 30 wt% or lower.

It is preferred to use the solvent in an amount 0.5 to 20 times by weight as much as the fresh onions, still preferably 1 to 10 times by weight and still more preferably 1 to 3 times by weight.

The extraction temperature is preferably 60°C or higher, still preferably 70°C or higher and particularly preferably 80°C or hither. It is preferred that the extraction is carried out for 30 minutes to 12 hours, still preferably 30 minutes to 6 hours and still more preferably 1 to 4 hours.

Examples of the procedure for removing the insoluble matters in the steps (a) to (c) include those commonly employed in the art, such as centrifugation, press filtration, paper filtration and filtration using a filter aid.
(d) A step of heating fresh onions and then cutting into small pieces, thereby giving a onion suspension extract or a concentrate thereof.

The step (d) is a step for obtaining an onion suspension, that is to be subjected to the distribution washing with an organic solvent, by cutting heated onions into small pieces to give an onion suspension and, if required, concentrating the same.

More specifically, an onion suspension or a concentrate thereof can be preferably obtained by, for example, the following process. First, fresh onions are heated until golden brown and, if required, a solvent is added thereto. Then, the onions are cut into small pieces with a food mixer, a homogenizer or the like.

Examples of the solvent to be added include water and aqueous ethanol. It is preferred that the ethanol concentration of the aqueous ethanol is 70 wt% or lower, still preferably 50 wt% or lower and particularly preferably 30 wt% or lower.

It is preferred to use the solvent in an amount 0.5 to 20 times by weight as much as the heated onions, still preferably 1 to 10 times by weight and particularly preferably 1 to 3 times by weight.

It is preferred to cut the heated onions to small pieces of 10 to 10,000 µm in size, still preferably 100 to 5,000 µm.

It is not required to remove the insoluble matters from the suspension obtained in the step (d).

The crude onion extract, onion suspension or concentrate thereof, which is obtained by any one of the aforesaid processes (a) to (d), is subjected to distribution washing using an organic solvent to thereby reduce the contents of quercetin and protocatechuic acid. If required, glutamylmethionine is added thereto. Thus, the onion extract of the invention can be obtained.

It is preferred to conduct the distribution washing with the organic solvent under the same conditions as the case of the distribution washing described above.

Thus, typical processes for obtaining the onion extract of the invention have been illustrated. These processes may be summarized as follows, i.e., (A) to (D).
(A) A process which includes at least either extracting or squeezing fresh onions to give a crude onion extract or a concentrate thereof, heating the same and then distribution washing with an organic solvent.
(B) A process which includes directly heating fresh onions, then at least either extracting or squeezing the same to give a crude onion extract or a concentrate thereof and then distribution washing with an organic solvent.
(C) A process which includes heat-extracting fresh onions (the extraction temperature being 60°C or higher and the extraction and heating being conducted simultaneously, thereby requiring no heat treatment thereafter), pressing the onions, if required, to give a crude onion extract or a concentrate thereof, and distribution washing with an organic solvent.
(D) A process which includes heating fresh onions, cutting into small pieces to give an onion suspension or a concentrate thereof, and then distribution washing with an organic solvent.

Although the crude onion extract or onion suspension is subjected to the solvent distribution washing at the final step in the processes (A) to (D), heat treatment may be conducted after the solvent distribution washing. Also, after the heated crude onion extract or onion suspension is then subjected to the solvent distribution washing, the onion extract thus obtained may be heated again.

In the invention, the procedure of treating the crude onion extract, concentrate thereof, onion suspension or concentrate thereof is not restricted to the aforesaid distribution washing with solvent, so long as the concentrations of glutamylmethionine, quercetin and protocatechuic acid in the solid matters in the onion extract can be controlled to the levels as defined above.

As the procedure for controlling the ingredients, other than distribution washing, an arbitrary procedure, such as a treatment with a cation exchange resin, a treatment with a synthetic resin adsorbent and so on, can be employed.

The onion extract of the invention can impart the flavor, deliciousness, sweetness, richness and so on of onion to foods or drinks without giving bitterness or astringency. Although there have been known various kinds of flavor products having onion flavors, the onion extract of the invention can be preferably used as an onion flavor ingredient capable of comprehensively presenting these onion flavors.

Typical examples of flavor products to which the onion extract of the invention can be added include onion flavors, beef flavors, pork flavors, chicken flavors, fried vegetable flavors and curry flavors.

Examples of other ingredients, which can be used together with the onion extract in flavor compositions, include alcohols, animal and vegetable fats and oils, polyhydric alcohols (propylene glycol, glycerol and so on), natural rubbers (gum arabic, gum tragacanth and so on), inclusion agents (cyclodextrin and so on), excipients (gelatin, dextrin and so on, being usable for powdering), carriers, stabilizers, colorants, antioxidants (vitamin E, vitamin C and so on), antimicrobial agents (benzoic acid, sodium benzoate and so on), shelf life-extending agent (sodium acetate, glycine and so on), preserving agents, extenders (anhydrous silicates, anhydrous sulfates, various inorganic chlorides, saccharides and polysaccharides), surfactants, pH-adjusting agents and other food additives.

Although the amount of the onion extract to be added to a flavor composition depends of flavor to be added, it is usually preferred to add 0.01 to 50 wt% of the onion extract relative to the total amount of the flavor composition.

As discussed above, the onion extract of the invention can impart the flavor, deliciousness, sweetness, richness and so on of onion to foods or drinks without giving bitterness or astringency. Therefore, it is preferably usable in foods or drinks in which onion has been employed as one of starting materials.

The onion extract of the invention may be directly added to foods or drinks. Alternatively, it may be added in the form of a flavor composition to foods or drinks. It is preferred to add 0.01 to 10 wt% of the flavor composition of the invention to foods or drinks.

The onion extract of the invention may be added either to half finished foods or drinks or to finished foods or drinks. Also, it may be added to foods or drinks in the form of dressings, grilled meat sauces and the like.

Specific examples of foods or drinks to which the onion extract of the invention can be added include roux and sauces of curry, hashed beef rice (*hayashi*), white stew and so on, dressings, marinade liquids, grilled meat sauces, *kabayaki* sauces, powdery soups, instant miso-soups, potage soups, corn soups, ramen soups, consomme soups, vegetable juices, sauces, meat products (hams, sausages and so on), snack foods, confectionary, prepared foods, frozen foods, health foods, soft drinks and so on, though the food or drink is not restricted thereto.

Also, the onion extract of the invention may be added to known seasonings, such as natural seasonings and compounded seasonings to use for flavoring foods or drinks.

Examples of the natural seasonings and compounded seasonings, to which the onion extract of the invention can be added, are as follows, though the invention is not restricted thereto.

### Natural seasonings:

Extracted type (meat extract, fish extract, vegetable extract and marine alga extract).
Digested type (yeast extract, hydrolyzed vegetable protein (HVP) and hydrolyzed animal protein (HAP))
Fermented type (soy source, miso, *mirin*, vinegar), compounded seasonings (prepared by adding taste-imparting seasonings, organic acids and saccharides to natural seasonings))

The onion extract of the invention may be added to a food or drink at any point in the process of producing the food or drink. If necessary, it may be added as a seasoning after the production of the food or drink and immediately before eating or drinking.

Although the amount of the onion extract of the invention in a food or drink depends on foods or drinks, it is usually preferred to add 0.00001 to 10 wt% of the onion extract to the food or drink.

### Examples

To illustrate the invention in greater detail, the following Examples will be given.

In Examples, the concentrations of the ingredients of glutamylmethionine (GM), quercetin (QC) and protocatechuic acid,(PA) were measured by the following methods.

### <Measurement of GM (glutamylmethionine) concentration>

100 ml of a 2 wt% solution of a sample (diluted with 70% EtOH) was passed through 30 ml of a strong acid cation exchange resin (AMBERLITE IR120BNa). The adsorbed fraction was eluted with aqueous ammonia, followed by concentrating and thereby removing ammonia. The resultant solution was subjected to derivatization with the use of ortho-phthalaldehyde. Then, the analysis was conducted under the following conditions.

### Measurement device: high performance liquid chromatograph (manufactured by Agilent)

Column: 5C₁₈-AR-II 4.6 x 150 mm (manufactured by Nakalai Tesque)
Column temperature: 40°C
Mobile phase: A: 40 mM sodium dihydrogen phosphate (pH 7.8)
   B: acetonitrile/methanol/water = 45/45/10
Gradient: A/B=100/0 → 70/30 (20 min) → 50/50 (30 min) → 50/50 (40 min)
Flow rate: 1.0 ml/min
Detector: UV absorptiometer (manufactured by Agilent)
Detection wavelength: 338 nm

### <Measurement of QC (quercetin) and PA (protocatechuic acid) concentrations>

An 8 wt% solution of a sample (diluted with 70% EtOH) was passed through a 0.45 µm syringe filter (manufactured by Whatman) to remove insoluble matters. Then, the analysis was conducted under the following conditions.

### Measurement device: high performance liquid chromatograph (manufactured by Agilent)

Column: CAPCELLPAK C₁₈ UG120 4.6 x 150 mm (manufactured by Shiseido Co., Ltd.)
Column temperature: 40°C
Mobile phase: A: 10 mM ammonium formate + 0.1 wt% formic acid
B: acetonitrile
Gradient: A/B= 90/10 → 90/10 (10 min) → 100/0 (40 min) → 100/0 (50 min)
Flow rate: 0.3 ml/min
Detector: UV absorptiometer (manufactured by Agilent)
Detection wavelength: 254 nm

### [Test Example 1]

According to the aforesaid measurement methods, the concentrations of glutamylmethionine (GM), quercetin (QC) and protocatechuic acid (PA) in the solid matters in a starting crude onion extract, that is a marketed product (manufactured by TOP FLAVORS) with Bx. 70, were measured. Table 1 shows the results.

**[Table 1]**

| | Concentration (ppm) in solid matters | | | QC/GM | PA/GM |
|---|---|---|---|---|---|
| | GM | QC | PA | | |
| Starting crude onion extract (marketed onion extract) | 193 | 814 | 743 | 4.22 | 3.85 |

### [Example 1]

750 g of ion-exchanged water was added to 250 g of the marketed starting crude onion extract (manufactured by TOP FLAVORS) used in Test Example 1, and the mixture was stirred at room temperature (25°C) for 1 hour. Next, 100 mL of ethyl acetate was added thereto and distribution washing was conducted. This washing procedure was repeated twice.

After the distribution washing, the washed matter was dissolved by adding an appropriate amount of ion-exchanged water thereto and the solution was vacuum concentrated until a pasty matter was obtained, thereby removing the remaining ethyl acetate. Thus, 232.5 g of onion extract A (Bx. 70) in a pasty form was obtained.

To 20 g of the onion extract A thus obtained, glutamylmethionine (GM) was added to adjust the glutamylmethionine concentration in the solid matters to 350 ppm. Thus, the onion extract of Example 1 was prepared. Table 2 shows the concentrations of the ingredients in the solid matters of the onion extract of Example 1.

### [Example 2]

To 20 g of the onion extract A obtained in Example 1, glutamylmethionine (GM) and quercetin (QC) were added so as to give concentrations in the solid matters of 350 ppm and 170 ppm, respectively. Thus, the onion extract of Example 2 was prepared. Table 2 shows the concentrations of the ingredients in the solid matters of the onion extract of Example 2.

### [Example 3]

To 20 g of the onion extract A obtained in Example 1, glutamylmethionine (GM) and protocatechuic acid (PA) were added so as to give concentrations in the solid matters of 350 ppm and 650 ppm, respectively. Thus, the onion extract of Example 3 was prepared. Table 2 shows the concentrations of the ingredients in the solid matters of the onion extract of Example 3.

### [Example 4]

To 20 g of the onion extract A produced in Example 1, glutamylmethionine (GM), quercetin (QC) and protocatechuic acid (PA) were added so as to give concentrations in the solid matters of 350 ppm, 170 ppm and 650 ppm, respectively. Thus, the onion extract of Example 4 was prepared. Table 2 shows the concentrations of the ingredients in the solid matters of the onion extract of Example 4.

### [Example 5]

To 20 g of the onion extract A produced in Example 1, glutamylmethionine (GM), quercetin (QC) and protocatechuic acid (PA) were added so as to give concentrations in the solid matters of 429 ppm, 170 ppm and 650 ppm, respectively. Thus, the onion extract of Example 5 was prepared. Table 2 shows the concentrations of the ingredients in the solid matters of the onion extract of Example 5.

### [Comparative Example 1]

The marketed starting crude onion extract (manufactured by TOP FLAVORS) used in Test Example 1 was employed as the onion extract of Comparative Example 1. Each of the concentrations of glutamylmethionine (GM), quercetin (QC) and protocatechuic acid (PA) in the solid matters of the marketed crude onion extract are as given in Test Example 1. By way of reference, Table 2 also shows these values.

### [Comparative Example 2]

The onion extract A produced in Example 1 was employed as the onion extract of Comparative Example 2. Each of the concentrations of glutamylmethionine (GM), quercetin (QC) and protocatechuic acid (PA) in the solid matters of onion extract A were measured by the aforesaid methods. Table 2 shows the results.

### [Comparative Example 3]

To 20 g of the onion extract A obtained in Example 1, glutamylmethionine (GM) and quercetin (QC) were added so as to give concentrations in the solid matters of 350 ppm and 214 ppm, respectively. Thus, the onion extract of Comparative Example 3 was prepared. Table 2 shows the concentrations of the ingredients in the solid matters of the onion extract of Comparative Example 3.

### [Comparative Example 4]

To 20 g of the onion extract A obtained in Example 1, glutamylmethionine (GM) and protocatechuic acid (PA) were added so as to give concentrations in the solid matters of 350 ppm and 714 ppm, respectively. Thus, the onion extract of Comparative Example 4 was prepared. Table 2 shows the concentrations of the ingredients in the solid matters of the onion extract of Comparative Example 4.

**[Table 2]**

| | Concentration (ppm) in solid matters | | | QC/GM | PA/GM |
|---|---|---|---|---|---|
| | GM | QC | PA | | |
| Extract of Ex. 1 | 350 | 129 | 600 | 0.369 | 1.71 |
| Extract of Ex. 2 | 350 | 170 | 600 | 0.486 | 1.71 |
| Extract of Ex. 3 | 350 | 129 | 650 | 0.369 | 1.86 |
| Extract of Ex. 4 | 350 | 170 | 650 | 0.486 | 1.86 |
| Extract of Ex. 5 | 429 | 170 | 650 | 0.396 | 1.52 |
| Extract of Comp. Ex. 1 | 193 | 814 | 743 | 4.22 | 3.85 |
| Extract of Comp. Ex. 2 | 229 | 129 | 600 | 0.563 | 2.62 |
| Extract of Comp. Ex. 3 | 350 | 214 | 600 | 0.611 | 1.71 |
| Extract of Comp. Ex. 4 | 350 | 129 | 714 | 0.369 | 2.04 |

### <Sensory Evaluation of Onion Extracts>

The concentrations of samples, i.e., the onion extracts of Examples 1 to 5 and Comparative Examples 1 to 4 were adjusted to Bx. 70 and the extracts themselves were sensorily evaluated without diluting or the like.

The evaluation was made on the onion flavor and harshness by 12 panelists. The flavor was evaluated in 3 grades (strong, weak and very weak), while the harshness was evaluated in 3 grades (strong, weak, no). Table 3 shows the results wherein numerical values represent the numbers of the panelists.

**[Table 3]**

| | Onion flavor | | | Harshness | | | Comment |
|---|---|---|---|---|---|---|---|
| | Strong | Weak | Very weak | Strong | Weak | No | |
| Example 1 | 10 | 2 | 0 | 0 | 2 | 10 | Strong onion flavor and very weak harshness |
| Example 2 | 9 | 3 | 0 | 0 | 4 | 8 | Strong onion flavor and somewhat weak harshness |
| Example 3 | 8 | 4 | 0 | 0 | 5 | 7 | Strong onion flavor and somewhat weak harshness |
| Example 4 | 8 | 4 | 0 | 0 | 6 | 6 | Strong onion flavor and weak harshness |
| Example 5 | 11 | 1 | 0 | 0 | 5 | 7 | Strong onion flavor and small harshness |
| Comparative Example 1 | 0 | 3 | 9 | 12 | 0 | 0 | Strong harshness and weak onion flavor |
| Comparative Example 2 | 0 | 11 | 1 | 0 | 3 | 9 | Weak onion flavor and weak harshness |
| Comparative Example 3 | 7 | 5 | 0 | 7 | 4 | 1 | Strong onion flavor and somewhat strong harshness |
| Comparative Example 4 | 6 | 6 | 0 | 8 | 4 | 0 | Strong onion flavor and strong harshness |

As Table 3 shows, the onion extracts of Examples 1 to 5, wherein the weight ratio of quercetin to glutamylmethionine (QC/GM) in the onion extract was from 0 to 0.49 and the weight ratio of protocatechuic acid to glutamylmethionine (PA/GM) in the onion extract was 0 to 1.86, showed strong onion flavor and small harshness.

In contrast thereto, the onion extracts of Comparative Examples 1 to 4, wherein the weight ratios QC/GM and/or PA/GM were outside of the ranges specified above, showed either or both of weak onion flavor and strong harshness.

These results indicate that an onion extract having strong onion flavor and small harshness can be obtained by controlling the weight ratio of quercetin to glutamylmethionine (QC/GM) in the onion extract to 0 to 0.49, and the weight ratio of protocatechuic acid to glutamylmethionine (PA/GM) in the onion extract to 0 to 1.86.

### [Example 6]

750 g of ion-exchanged water was added to 250 g of the marketed starting crude onion extract product (manufactured by TOP FLAVORS) used in Test Example 1, and the mixture was stirred at 100°C for 1 hour. Then, the mixture was cooled until the internal temperature attained room temperature to give a crude onion extract.

89 g of ethyl acetate was added to 990 g of the crude onion extract, and distribution washing was conducted. This washing procedure was repeated twice. After the distribution washing, the washed extract was vacuum concentrated until a pasty matter thereof was obtained. Then, ethanol was added in an amount 1.25 times by weight as much as the concentrated extract and distribution washing was conducted. The distribution washing procedure was repeated twice. After adding a small amount of ion-exchanged water to the washed matter, the mixture was vacuum concentrated to thereby remove the remaining ethanol. Thus, 97 g of the onion extract of Example 6 (Bx. 70) was obtained.

The concentrations of glutamylmethionine (GM), quercetin (QC) and protocatechuic acid (PA) in the solid matters of the onion extract thus obtained were measured by the aforesaid methods. Table 4 shows the results.

### [Comparative Example 5]

750 g of ion-exchanged water was added to 250 g of the marketed starting crude onion extract product (manufactured by TOP FLAVORS) used in Test Example 1, and the mixture was stirred at room temperature (25°C) for 1 hour. This crude onion extract was vacuum concentrated until a pasty matter thereof was obtained. Thus, 241 g of the onion extract of Comparative Example 5 (Bx. 70) was obtained.

The concentrations of glutamylmethionine (GM), quercetin (QC) and protocatechuic acid (PA) in the solid matters of the onion extract thus obtained were measured by the aforesaid methods. Table 4 shows the results.

### [Comparative Example 6]

750 g of ion-exchanged water was added to 250 g of the marketed starting crude onion extract product (manufactured by TOP FLAVORS) used in Test Example 1, and the mixture was stirred at 100°C for 1 hour. After cooling the mixture until the internal temperature attained room temperature, the mixture was vacuum concentrated until a pasty matter thereof was obtained. Thus, 240 g of the onion extract of Comparative Example 6 (Bx. 70) was obtained.

The concentrations of glutamylmethionine (GM), quercetin (QC) and protocatechuic acid (PA) in the solid matters of the onion extract thus obtained were measured by the aforesaid methods. Table 4 shows the results.

### [Comparative Example 7]

750 g of ion-exchanged water was added to 250 g of the marketed starting crude onion extract product (manufactured by TOP FLAVORS) used in Test Example 1, and the mixture was stirred at room temperature (25°C) for 1 hour. After stirring, 89 g of ethyl acetate was added and distribution washing was conducted. This washing procedure was repeated twice.

The washed onion extract was vacuum concentrated until a pasty matter thereof was obtained. Then, ethanol was added in an amount 1.25 times by weight as much as the concentrated extract and distribution washing was conducted. The distribution washing procedure was repeated twice. After adding a small amount of ion-exchanged water to the washed matter, the mixture was vacuum concentrated to thereby remove the remaining ethanol, Thus, 103 g of the onion extract of Comparative Example 7 (Bx. 70) was obtained.

The concentrations of glutamylmethionine (GM), quercetin (QC) and protocatechuic acid (PA) in the solid matters of the onion extract thus obtained were measured by the aforesaid methods. Table 4 shows the results.

**[Table 4]**

| | Concentration (ppm) in solid matters | | | QC/GM | PA/GM |
|---|---|---|---|---|---|
| | GM | QC | PA | | |
| Extract of Ex. 6 | 516 | 29 | 560 | 0.0562 | 1.09 |
| Extract of Comp.Ex. 5 | 194 | 811 | 742 | 4.18 | 3.82 |
| Extract of Comp.Ex. 6 | 287 | 809 | 728 | 2.82 | 2.54 |
| Extract of Comp.Ex. 7 | 240 | 29 | 555 | 0.121 | 2.31 |

### <Sensory Evaluation of Onion Extracts>

The onion extracts obtained in Example 6 and Comparative Examples 5 to 7 were sensorily evaluated by 3 panelists according to the following method. The evaluation items included the sweetness, umami, richness, astringency, bitterness, sourness and burnt flavor of the onion extracts. The average was calculated for each evaluation item. Table 5 shows the results.

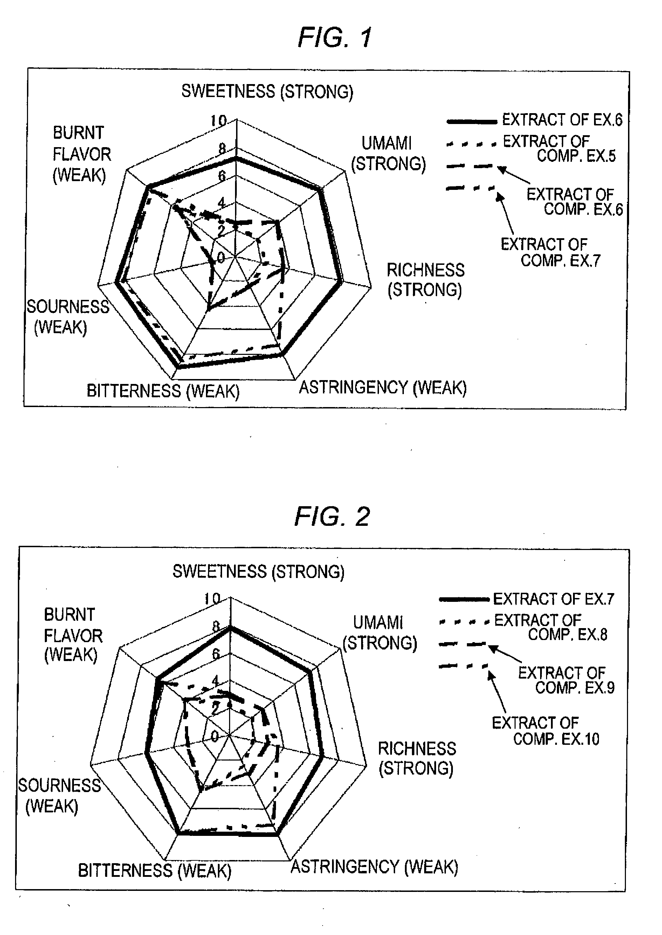

The cobweb chart in Fig. 1 also shows the results. In Fig. 1, data points of higher grade are more distant from the center in respect of sweetness, umami and richness, while data points of lower grade are more distant from the center in respect of astringency, bitterness, sourness and burnt flavor.

### (Evaluation method)

Each onion extract sample (Bx. 70) was diluted 15-fold with distilled water. The panelists held the diluted sample in the mouth and absolutely evaluated the individual items for sensory evaluation (sweetness, umami, richness, astringency, bitterness, sourness and burnt flavor) in 10 grades (1 to 10). Sweetness, umami and richness were evaluated in an ascending manner, i.e., 1 (weak) to 10 (strong), whereas astringency, bitterness, sourness and burnt flavor were evaluated in a descending manner, i.e., 1 (strong) to 10 (weak).

**[Table 5]**

| | Sweetness (strong) | Umami (strong) | Richness (strong) | Astringency (weak) | Bitterness (weak) | Sourness (weak) | Burnt flavor (weak) |
|---|---|---|---|---|---|---|---|
| Extract of Ex.6 | 7.2 | 7.7 | 7.7 | 8.0 | 9.0 | 8.7 | 8.0 |
| Extract of Comp.Ex.5 | 2.3 | 2.0 | 2.3 | 2.3 | 4.3 | 1.7 | 5.3 |
| Extract of Comp.Ex.6 | 2.5 | 3.9 | 3.5 | 2.4 | 4.3 | 1.6 | 5.6 |
| Extract of Comp.Ex.7 | 2.4 | 3.8 | 3.5 | 7.2 | 8.4 | 8.1 | 7.6 |

As Table 5 and Fig. 1 show, the onion extract of Example 6 that is the onion extract of the invention showed extremely stronger sweetness, umami and richness than those of Comparative Examples.

The onion extract of Example 6 that is the onion extract of the invention showed weaker astringency, bitterness, sourness and burnt flavor than those of Comparative Examples.

These results indicate that the onion extract of the invention is a highly excellent taste-imparting agent capable of imparting onion flavor.

### [Example 7]

500 g of marketed onions (produced in Hokkaido), which had been peeled and cut into small pieces, were heated until golden brown on a frying pan heated on a stove burner. 1000 g of ion-exchanged water was added to 470 g of the heated onions, and the onions were extracted under stirring at 30°C for 4 hours. After removing the insoluble matters by passing through a filter paper, 1180 g of a clear liquid was obtained. Next, it was vacuum concentrated to give 53 g of a crude onion extract with Bx. 70.

30 g of ethyl acetate was added to this crude onion extract, and distribution washing was conducted. This washing procedure was repeated twice. 20 g of ion-exchanged water was added to the extract having been treated with ethyl acetate, and the mixture was vacuum concentrated to remove the remaining ethyl acetate. Thus, 37 g of an onion extract with Bx. 70, which was referred to as the onion extract of Example 7, was obtained.

The concentrations of glutamylmethionine (GM), quercetin (QC) and protocatechuic acid (PA) in the solid matters of the onion extract thus obtained were measured by the aforesaid methods. Table 6 shows the results.

### [Comparative Example 8]

1000 g of ion-exchanged water was added to 500 g of marketed onions (produced in Hokkaido), which had been peeled and cut into small pieces, and the onions were extracted under stirring at 30°C for 4 hours. After removing the insoluble matters by passing through a filter paper, 1060 g of a clear liquid was obtained. Next, it was vacuum concentrated to give 50 g of a crude onion extract with Bx. 70 which was referred to as the onion extract of Comparative Example 8.

The concentrations of glutamylmethionine (GM), quercetin (QC) and protocatechuic acid (PA) in the solid matters of the onion extract thus obtained were measured by the aforesaid methods. Table 6 shows the results.

### [Comparative Example 9]

500 g of marketed onions (produced in Hokkaido), which had been peeled and cut into small pieces, were heated until golden brown in a frying pan heated on a stove burner. 1000 g of ion-exchanged water was added to 470 g of the heated onions, and the onions were extracted under stirring at 30°C for 4 hours. After removing the insoluble matters by passing through a filter paper, 1180 g of a clear liquid was obtained. Next, it was vacuum concentrated to give 53 g of a crude onion extract with Bx. 70 which was referred to as the onion extract of Comparative Example 9.

The concentrations of glutamylmethionine (GM), quercetin (QC) and protocatechuic acid (PA) in the solid matters of the onion extract thus obtained were measured by the aforesaid methods. Table 6 shows the results.

### [Comparative Example 10]

1000 g of ion-exchanged water was added to 500 g of marketed onions (produced in Hokkaido), which had been peeled and cut into small pieces, and the onions were extracted under stirring at 30°C for 4 hours. After removing the insoluble matters by passing through a filter paper, 1060 g of a clear liquid was obtained. Next, it was vacuum concentrated to give 50 g of a crude onion extract with Bx. 70.

28 g of ethyl acetate was added to this crude onion extract, and distribution washing was conducted. This washing procedure was repeated twice. 15 g of ion-exchanged water was added to the extract having been treated with ethyl acetate, and the mixture was vacuum concentrated. Thus, 21 g of a concentrated extract with Bx. 70, which was referred to as the onion extract of Comparative Example 10, was obtained.

The concentrations of glutamylmethionine (GM), quercetin (QC) and protocatechuic acid (PA) in the solid matters of the onion extract thus obtained were measured by the aforesaid methods. Table 6 shows the results.

**[Table 6]**

| | Concentration (ppm) in solid matters | | | QC/GM | PA/GM |
|---|---|---|---|---|---|
| | GM | QC | PA | | |
| Extract of Ex. 7 | 373 | 58 | 330 | 0.155 | 0.885 |
| Extract of Comp.Ex. 8 | 116 | 385 | 432 | 3.32 | 3.72 |
| Extract of Comp.Ex. 9 | 192 | 388 | 440 | 2.02 | 2.29 |
| Extract of Corap.Ex. 10 | 120 | 58 | 333 | 0.483 | 2.78 |

### <Sensory Evaluation of Onion Extracts>

According to the aforesaid evaluation method, the onion extracts obtained in Example 7 and Comparative Examples 8 to 10 were sensorily evaluated by 5 panelists. The evaluation items included the sweetness, umami, richness, astringency, bitterness, sourness and burnt flavor of the onion extracts. The average was calculated for each evaluation item. Table 7 shows the results.

The cobweb chart in Fig. 2 also shows the results. In Fig. 2, data points of higher grade are more distant from the center in respect of sweetness, umami and richness, while data points of lower grade are more distant from the center in respect of astringency, bitterness, sourness and burnt flavor.

**[Table 7]**

| | Sweetness (strong) | Umami (strong) | richness (strong) | Astringency (weak) | Bitterness (weak) | Sourness (weak) | Burnt flavor (weak) |
|---|---|---|---|---|---|---|---|
| Extract of Example 7 | 7.8 | 7.3 | 6.8 | 8.0 | 7.9 | 5.9 | 6.5 |
| Extract of Comparative Example 8 | 2.3 | 2.0 | 1.8 | 2.5 | 4.3 | 3.0 | 4.0 |
| Extract of Comparative Example 9 | 2.8 | 3.0 | 2.8 | 3.1 | 4.5 | 3.0 | 4.1 |
| Extract of Comparative Example 10 | 3.0 | 3.0 | 3.5 | 7.2 | 7.8 | 5.9 | 6.1 |

As Table 7 and Fig. 2 show, the onion extract of Example 7 that is the onion extract of the invention showed extremely stronger sweetness, umami and richness than those of Comparative Examples.

The astringency, bitterness, sourness and burnt flavor of the onion extract of Example 7, that is the onion extract of the invention, were each equivalent to or weaker than those of Comparative Examples.

These results indicate that the onion extract of the invention is a highly excellent taste-imparting agent capable of imparting onion flavor.

### [Example 8]

800 g of 20 wt% aqueous ethanol was added to 400 g of marketed onions (produced in Hokkaido), which had been peeled and cut into small pieces. Next, the mixture was heated to 80°C, followed by extracting for 2 hours under stirring. After removing the insoluble matters by passing through a filter paper, the filtrate was vacuum concentrated to give 85 g of a crude onion extract with Bx. 65.

40 g of ethyl acetate was added to this crude onion extract, and distribution washing was conducted. 20 g of ion-exchanged water was added to the washed matter, and the mixture was vacuum concentrated. Thus, 60 g of a crude onion extract with Bx. 70 was obtained. Then, 60 g of ethanol was added to the crude onion extract and distribution washing was conducted. 20 g of ion-exchanged water was added to the washed matter, and the mixture was vacuum concentrated. Thus, 48 g of an onion extract with Bx. 70, which was referred to as the onion extract of Example 8, was obtained.

The concentrations of glutamylmethionine (GM), quercetin (QC) and protocatechuic acid (PA) in the solid matters of the onion extract thus obtained were measured by the aforesaid methods. Table 8 shows the results.

**[Table 8]**

| | Concentration (ppm) in solid matters | | | QC/GM | PA/GM |
|---|---|---|---|---|---|
| | GM | QC | PA | | |
| Extract of Example 8 | 474 | 57 | 430 | 0.120 | 0.907 |

### [Example 9]

### <Sensory Evaluation of Onion Extract-Containing Foods or Drinks>

The onion extract of Example 8 was added to the following foods or drinks. To compare the flavors of the foods or drinks before and after the addition, sensory evaluation was conducted. The sensory evaluation was conducted by 4 panelists.

### • Curry

15 g of a marketed solid curry roux "BAMONTO CURRY® (Sweet)" (House Foods Corp.) was added to 120 g of water, and the mixture was dissolved by heating to give a curry sauce. Then, 0.1 wt% of the onion extract of Example 8 was added to the curry sauce to give an onion extract-containing curry sauce.

### Evaluation Results

Compared with the curry sauce to which the onion extract was not added, the sweetness of onion was imparted to the onion extract-containing curry sauce so that the deliciousness as curry was improved.

### • Hashed Beef Rice (hayashi)

20 g of a marketed solid *hayashi* roux BAMONTO HAYASHI® (House Foods Corp.) was added to 110 g of water, and the mixture was dissolved by heating to give a *hayashi* sauce. Then, 0.1 wt% of the onion extract of Example 8 was added to the *hayashi* sauce to give an onion extract-containing *hayashi* sauce.

### Evaluation Results

Compared with the *hayashi* sauce to which the onion extract was not added, the onion extract-containing *hayashi* sauce showed improved flavor of onion and enriched thickness of *hayashi* sauce.

### • White Stew

15 g of a marketed stew roux "HOKKAIDO STEW (Cream)" (House Foods Corp.) was added to 120 g of water and 20 g of marketed milk "MEIJI OISHII GYUNYU" (Meiji Dairies Corporation), and the mixture was dissolved by heating to give a white stew sauce. Then, 0.05 wt% of the onion extract of Example 8 was added to the white stew sauce to give an onion extract-containing white stew sauce.

### Evaluation Results

Compared with the white stew sauce to which the onion extract was not added, the sweetness of onion was imparted to the onion extract-containing white stew sauce so that the deliciousness as white stew was improved.

### • Dressing

0.06 wt% of the onion extract of Example 8 was added to marketed dressing "SARADA WO OISHIKU TABERU OSU MAROYAKA TOMATO-SU" (Mizkan), thereby giving an onion extract-containing dressing.

### Evaluation Results

Compared with the dressing to which the onion extract was not added, the sweetness of onion was imparted to the onion extract-containing dressing and sourness was relieved.

### • Marinade Liquid

0.08 wt% of the onion extract of Example 8 was added to marketed marinade liquid "MARINE-YO" (Kewpie Corporation), thereby giving an onion extract-containing marinade liquid.

### Evaluation Results

Compared with the marinade liquid to which the onion extract was not added, the sweetness of onion was imparted to the onion extract-containing marinade liquid and sourness was relieved.

### • Grilled Meat Sauce

0.15 wt% of the onion extract of Example 8 was added to marketed grilled meat sauce "OGON NO AJI® Mild-hotness" (Ebara Food Industry Inc.), thereby giving an onion extract-containing grilled meat sauce.

### Evaluation Results

Compared with the grilled meat sauce to which the onion extract was not added, the sweetness of onion was imparted to the onion extract-containing grilled meat sauce so that the deliciousness as grilled meat sauce was improved.

### • Kabayaki Sauce

0.3 wt% of the onion extract of Example 8 was added to marketed *kabayaki* sauce "KABAYAKI NO TARE" (Suzukatsu Co., Ltd.), thereby giving an onion extract-containing *kabayaki* sauce.

### Evaluation Results

Compared with the *kabayaki* sauce to which the onion extract was not added, the sweetness of onion was imparted to the onion extract-containing *kabayaki* sauce so that the deliciousness as *kabayaki* sauce was improved.

### • Instant Miso-Soup

19 g of marketed instant miso-soup "ASAGE (NAMA MISO TYPE)®" (Nagatanien Co., Ltd.) was added to 180 g of hot water and the mixture was dissolved by stirring to give instant miso-soup. 0.02 wt% of the onion extract of Example 8 was added to this miso-soup, thereby giving an onion extract-containing miso-soup.

### Evaluation Results

Compared with the miso-soup to which the onion extract was not added, the sweetness of onion was imparted to the onion extract-containing miso-soup so that the deliciousness as miso-soup was improved.

### • Potage Soup

19.8 g of marketed potage soup "KUNORU HOKKAIDO TORORI POTAGE (dry soup powder)" (AJINOMOTO CO., INC.) was added to 150 g of hot water and the mixture was dissolved by stirring to give potage soup. 0,1 wt% of the onion extract of Example 8 was added to this potage soup, thereby giving an onion extract-containing potage soup.

### Evaluation Results

Compared with the potage soup to which the onion extract was not added, the umami of onion was imparted to the onion extract-containing potage soup so that the deliciousness as soup was improved.

### • Corn Soup

19.6 g of marketed corn soup "KUNORU KAPPU SOUP CORN CREAM (dry soup powder)" (AJINOMOTO CO., INC.) was added to 150 g of hot water and dissolved by stirring to give corn soup. 0.05 wt% of the onion extract of Example 8 was added to this corn soup, thereby giving an onion extract-containing corn soup.

### Evaluation Results

Compared with the corn soup to which the onion extract was not added, the umami of onion was imparted to the onion extract-containing corn soup so that the deliciousness as soup was improved.

### • Ramen Soup

Powdery soup (8.5 g), which was packaged with marketed instant Chinese noodles "SAPPORO ICHIBAN® (soy source taste)" (SANYO FOODS CO., LTD.), was dissolved in 500 g of hot water to give ramen soup. 0.03 wt% of the onion extract of Example 8 was added to this ramen soup, thereby giving an onion extract-containing Chinese noodle soup.

### Evaluation Results

Compared with the ramen soup to which the onion extract was not added, the sweetness of onion was imparted to the onion extract-containing ramen soup so that the deliciousness as ramen soup was improved.

### • Consomme Soup

5.3 g of marketed dry consomme soup "AJINOMOTO KK CONSOMME (Solid Type)" (AJINOMOTO CO., INC.) was added to 300 g of water and the mixture was dissolved by heating to give consomme soup. 0.04 wt% of the onion extract of Example 8 was added to this consomme soup, thereby giving an onion extract-containing consomme soup.

### Evaluation Results

Compared with the consomme soup to which the onion extract was not added, the sweetness of onion was imparted to the onion extract-containing consomme soup so that the deliciousness as soup was improved.

### • Vegetable Juice

0.02 wt% of the onion extract of Example 8 was added to marketed vegetable juice "ICHINICHI-BUN NO YASAI VEGETABLE 100" (ITO EN, LTD.), thereby giving an onion extract-containing vegetable juice.

### Evaluation Results

Compared with the vegetable juice to which the onion extract was not added, the sweetness of onion was imparted to the onion extract-containing vegetable juice so that the deliciousness as vegetable juice was improved.

### [Example 10]

20 wt% of the onion extract of Example 8, 0.001 wt% of onion essential oil "ONION OIL" (Plus One), 30 wt% of glycerin, 0.999 wt% of ethanol and 49 wt% of water were mixed together to give a flavor composition of Example 10.

### [Comparative Example 11]

0.001 wt% of onion essential oil "ONION OIL" (Plus One), 30 wt% of glycerin, 0.999 wt% of ethanol and 69 wt% of water were mixed together to give a flavor composition of Comparative Example 11.

### [Comparative Example 12]

20 wt% of the starting crude onion extract of Test Example 1, 0.001 wt% of onion essential oil "ONION OIL" (Plus One), 30 wt% of glycerin, 0.999 wt% of ethanol and 49 wt% of water were mixed together to give a flavor composition of Comparative Example 12.

Table 9 summarizes the composition of the flavor compositions prepared in the above Example 10 and Comparative Examples 11 and 12.

**[Table 9]**

| | Onion extract of Example 8 | Starting crude onion extract of Test Example 1 | Onion essential oil | Glycerin | Ethanol | Water |
|---|---|---|---|---|---|---|
| Flavor composition of Example 10 | 20 | - | 0.001 | 30 | 0.999 | 49 |
| Flavor composition of Comparative Example 11 | - | - | 0.001 | 30 | 0.999 | 69 |
| Flavor composition of Comparative Example 12 | - | 20 | 0.001 | 30 | 0.999 | 49 |

| | | | | | | |
|---|---|---|---|---|---|---|
| Numerical values in the table are expressed in wt%. Onion essential oil: ONION OIL (Plus One) | | | | | | |

### <Sensory Evaluation of Flavor composition-Containing Dressings>

Using a marketed dressing "SARADA WO OISHIKU TABERU OSU MAROYAKA TOMATO-SU" (Mizkan) as a base, 0.1 wt% of the flavor compositions of Example 10, Comparative Example 11 and Comparative Example 12 were added, respectively, to thereby give a dressing containing the flavor composition of Example 10, a dressing containing the flavor composition of Comparative Example 11 and a dressing containing the flavor composition of Comparative Example 12. Then, the flavors of these flavor composition-containing dressings were compared. The sensory evaluation was conducted by 6 panelists. Table 10 shows the results of the sensory evaluation.

**[Table 10]**

| | Sweetness | | | Sourness | | | Comment |
|---|---|---|---|---|---|---|---|
| | Strong | Weak | No | Strong | Weak | No | |
| Dressing containing flavor composition of Example 10 | 6 | 0 | 0 | 0 | 1 | 5 | Sweetness is imparted to dressing and irritating sourness is relieved |
| Dressing containing flavor composition of Comparative Example 11 | 0 | 1 | 5 | 4 | 2 | 0 | Onion flavor is enriched but sweetness and sourness are unchanged |
| Dressing containing flavor composition of Comparative Example 12 | 1 | 4 | 1 | 2 | 3 | 1 | Sweetness and flavor are enriched but harshness is noticeable |

As the comments in Table 10 indicate, the dressing containing the flavor composition of Example 10 showed enriched sweetness and relieved irritating sourness. It also showed improved deliciousness as dressing.

In contrast thereto, the dressing containing the flavor composition of Comparative Example 11 showed enriched onion flavor but the sweetness and irritating sourness of this dressing were unchanged. The dressing containing the flavor composition of Comparative Example 12 showed little enriched sweetness and noticeable harshness.

This application is based on Japanese Patent Application No. 2009-168394 filed on July 17, 2009.

## Claims

1. An onion extract which contains glutamylmethionine, quercetin and protocatechuic acid, wherein a weight ratio of the quercetin to the glutamylmethionine (quercetin/glutamylmethionine) is 0 to 0.49 and a weight ratio of the protocatechuic acid to the glutamylmethionine (protocatechuic acid/glutamylmethionine) is 0 to 1.86.

2. The onion extract according to claim 1, wherein a concentration of the glutamylmethionine in an extract solid matter is 350 ppm or more, a concentration of the quercetin in the extract solid matter is 170 ppm or less, and a concentration of the protocatechuic acid in the extract solid matter is 650 ppm or less.

3. The onion extract according to claim 1 or 2, which is obtainable by a production process comprising: any one of the following steps (a) to (d); and a step of distribution washing a crude onion extract, onion suspension or concentrate thereof, which has been obtained by the following step, with an organic solvent,
(a) a step of heating the crude onion extract or concentrate thereof, which is obtained by at least either extracting or squeezing a fresh onion;
(b) a step of heating a fresh onion, and then obtaining the crude onion extract or concentrate thereof by at least either extracting or squeezing;
(c) a step of heat-extracting a fresh onion, thereby giving the crude onion extract or concentrate thereof; and
(d) a step of heating a fresh onion and then cutting into small pieces, thereby giving the onion suspension or concentrate thereof.

4. The onion extract according to claim 3, wherein the organic solvent used in the distribution washing is at least one of ethyl acetate and ethanol.

5. A process for producing an onion extract, the process comprising: any one of the following steps (a) to (d); and a step of distribution washing a crude onion extract, onion suspension or concentrate thereof, which has been obtained by the following step, with an organic solvent, wherein the onion extract contains glutamylmethionine, quercetin and protocatechuic acid, and a weight ratio of the quercetin to the glutamylmethionine (quercetin/glutamylmethionine) is 0 to 0.49 and a weight ratio of the protocatechuic acid to the glutamylmethionine (protocatechuic acid/glutamylmethionine) is 0 to 1.86,
(a) a step of heating the crude onion extract or concentrate thereof, which is obtained by at least either extracting or squeezing a fresh onion;
(b) a step of heating a fresh onion, and then obtaining the crude onion extract or concentrate thereof by at least either extracting or squeezing;
(c) a step of heat-extracting a fresh onions, thereby giving the crude onion extract or concentrate thereof; and
(d) a step of heating a fresh onion and then cutting into small pieces, thereby giving the onion suspension or concentrate thereof.

6. The production process according to claim 5, wherein
the organic solvent used in the distribution washing is at least one of ethyl acetate and ethanol.

7. A flavor composition, which contains 0.01 to 50 wt% of the onion extract according to any of claims 1 to 4.

8. A food or drink, which contains 0.01 to 10 wt% of the flavor composition according to claim 7.

9. A food or drink which contains 0.00001 to 10 wt% of the onion extract according to any of claims 1 to 4.

## Patentansprüche

1. Zwiebelextrakt, welcher Glutamylmethionin, Quercetin und Protocatechusäure enthält, wobei ein Gewichtsverhältnis des Quercetins zu dem Glutamylmethionin (Quercetin/Glutamylmethionin) 0 bis 0,49 beträgt und ein Gewichtsverhältnis der Protocatechusäure zu dem Glutamylmethionin (Protocatechusäure/Glutamylmethionin) 0 bis 1,86 beträgt.

2. Zwiebelextrakt nach Anspruch 1, wobei eine Konzentration des Glutamylmethionins in einem Extraktfeststoff 350 ppm oder mehr beträgt, eine Konzentration des Quercetins in dem Extraktfeststoff 170 ppm oder weniger beträgt und eine Konzentration der Protocatechusäure in dem Extraktfeststoff 650 ppm oder weniger beträgt.

3. Zwiebelextrakt nach Anspruch 1 oder 2, welcher erhältlich ist durch ein Herstellungsverfahren, umfassend: einen der folgenden Schritte (a) bis (d); und einen Schritt der Verteilungs-Waschung eines rohen Zwiebelextrakts, einer Zwiebelsuspension oder eines Konzentrats davon, welcher bzw. welche bzw. welches durch den folgenden Schritt erhalten wurde, mit einem organischen Lösungsmittel,
(a) einen Schritt des Erhitzens des rohen Zwiebelextrakts oder Konzentrats davon, welcher bzw. welches erhalten wird durch wenigstens entweder Extrahieren oder Auspressen einer frischen Zwiebel;
(b) einen Schritt des Erhitzens einer frischen Zwiebel und anschließend des Erhaltens des rohen Zwiebelextrakts oder Konzentrats davon durch wenigstens entweder Extrahieren oder Auspressen;
(c) einen Schritt des Heißextrahierens einer frischen Zwiebel, wodurch der rohe Zwiebelextrakt oder das Konzentrat davon erhalten wird; und
(d) einen Schritt des Erhitzens einer frischen Zwiebel und anschließend des Schneidens in kleine Stücke, wodurch die Zwiebelsuspension oder das Konzentrat davon erhalten wird.

4. Zwiebelextrakt nach Anspruch 3, wobei das in der Verteilungs-Waschung verwendete organische Lösungsmittel wenigstens eines von Ethylacetat und Ethanol ist.

5. Verfahren zum Herstellen eines Zwiebelextrakts, wobei das Verfahren umfasst: einen der folgenden Schritte (a) bis (d); und einen Schritt der Verteilungs-Waschung eines rohen Zwiebelextrakts, einer Zwiebelsuspension oder eines Konzentrats davon, welcher bzw. welche bzw. welches durch den folgenden Schritt erhalten wurde, mit einem organischen Lösungsmittel, wobei der Zwiebelextrakt Glutamylmethionin, Quercetin und Protocatechusäure enthält und ein Gewichtsverhältnis des Quercetins zu dem Glutamylmethionin (Quercetin/Glutamylmethionin) 0 bis 0,49 beträgt und ein Gewichtsverhältnis der Protocatechusäure zu dem Glutamylmethionin (Protocatechusäure/Glutamylmethionin) 0 bis 1,86 beträgt,
(a) einen Schritt des Erhitzens des rohen Zwiebelextrakts oder Konzentrats davon, welcher bzw. welches erhalten wird durch wenigstens entweder Extrahieren oder Auspressen einer frischen Zwiebel;
(b) einen Schritt des Erhitzens einer frischen Zwiebel und anschließend des Erhaltens des rohen Zwiebelextrakts oder Konzentrats davon durch wenigstens entweder Extrahieren oder Auspressen;
(c) einen Schritt des Heißextrahierens einer frischen Zwiebel, wodurch der rohe Zwiebelextrakt oder das Konzentrat davon erhalten wird; und
(d) einen Schritt des Erhitzens einer frischen Zwiebel und anschließend des Schneidens in kleine Stücke, wodurch die Zwiebelsuspension oder das Konzentrat davon erhalten wird.

6. Herstellungsverfahren nach Anspruch 5, wobei das in der Verteilungs-Waschung verwendete organische Lösungsmittel wenigstens eines von Ethylacetat und Ethanol ist.

7. Aromazusammensetzung, welche 0,01 bis 50 Gew.-% des Zwiebelextrakts nach einem der Ansprüche 1 bis 4 enthält.

8. Lebensmittel oder Getränk, welches 0,01 bis 10 Gew.-% der Aromazusammensetzung nach Anspruch 7 enthält.

9. Lebensmittel oder Getränk, welches 0,00001 bis 10 Gew.-% des Zwiebelextrakts nach einem der Ansprüche 1 bis 4 enthält.

## Revendications

1. Extrait d'oignon qui contient de la glutamylméthionine, de la quercétine et de l'acide protocatéchuique, dans lequel un rapport en poids de la quercétine sur la glutamylméthionine (quercétine/glutamylméthionine) est compris entre 0 et 0,49 et un rapport en poids de l'acide protocatéchuique sur la glutamylméthionine (acide protocatéchuique/glutamylméthionine) est compris entre 0 et 1,86.

2. Extrait d'oignon selon la revendication 1, dans lequel une concentration de la glutamylméthionine dans un extrait de matière solide est supérieure ou égale à 350 ppm, une concentration de la quercétine dans l'extrait de matière solide est inférieure ou égale à 170 ppm et une concentration de l'acide protocatéchuique dans l'extrait de matière solide est inférieure ou égale à 650 ppm.

3. Extrait d'oignon selon la revendication 1 ou 2, qui peut être obtenu par un procédé de production comprenant : l'une quelconque des étapes (a) à (d) suivantes ; et une étape de lavage par diffusion d'un extrait d'oignon brut, d'une suspension d'oignon ou d'un concentré de ce dernier, qui a été obtenu par l'étape suivante, avec un solvant organique,
(a) une étape de chauffage de l'extrait d'oignon brut ou du concentré de ce dernier, qui est obtenu au moins par extraction ou pressage d'un oignon frais ;
(b) une étape de chauffage d'un oignon frais, puis d'obtention de l'extrait d'oignon brut ou du concentré de ce dernier au moins par extraction ou pressage ;
(c) une étape d'extraction à chaud d'un oignon frais, conduisant ainsi à l'extrait d'oignon brut ou concentré de ce dernier ; et
(d) une étape de chauffage d'un oignon frais puis de découpe en petits morceaux, conduisant ainsi à la suspension d'oignon ou au concentré de ce dernier.

4. Extrait d'oignon selon la revendication 3, dans lequel le solvant organique utilisé dans le lavage par diffusion est au moins l'un parmi l'acétate d'éthyle et l'éthanol.

5. Procédé de production d'un extrait d'oignon, le procédé comprenant : l'une quelconque des étapes (a) à (d) suivantes ; et une étape de lavage par diffusion d'un extrait d'oignon brut, d'une suspension d'oignon ou d'un concentré de ce dernier, qui a été obtenu par l'étape suivante, avec un solvant organique, dans lequel l'extrait d'oignon contient de la glutamylméthionine, de la quercétine et de l'acide protocatéchuique, et un rapport en poids de la quercétine sur la glutamylméthionine (quercétine/glutamylméthionine) est compris entre 0 et 0,49 et un rapport de poids de l'acide protocatéchuique sur la glutamylméthionine (acide protocatéchuique/glutamylméthionine) est compris entre 0 et 1,86,
(a) une étape de chauffage de l'extrait d'oignon brut ou du concentré de ce dernier, qui est obtenu au moins par extraction ou pressage d'un oignon frais ;
(b) une étape de chauffage d'un oignon frais, puis d'obtention de l'extrait d'oignon brut ou du concentré de ce dernier au moins par extraction ou pressage ;
(c) une étape d'extraction à chaud d'un oignon frais, conduisant ainsi à l'extrait d'oignon brut ou au concentré de ce dernier ; et
(d) une étape de chauffage d'un oignon frais puis de découpe en petits morceaux, conduisant ainsi à la suspension d'oignon ou au concentré de ce dernier.

6. Procédé de production selon la revendication 5, dans lequel le solvant organique utilisé dans le lavage par diffusion est au moins l'un parmi l'acétate d'éthyle et l'éthanol.

7. Composition d'arôme qui contient de 0,01 à 50 % en poids de l'extrait d'oignon selon l'une quelconque des revendications 1 à 4.

8. Aliment ou boisson qui contient de 0,01 à 10 % en poids de la composition d'arôme selon la revendication 7.

9. Aliment ou boisson qui contient de 0,00001 à 10 % en poids de l'extrait d'oignon selon l'une quelconque des revendications 1 à 4.
